# EUROPEAN PATENT APPLICATION

(11) **EP 3 305 282 A2**
(43) Date of publication of application: **11.04.2018**
(21) Application number: 16803732.3
(22) Date of filing: 01.06.2016
(51) Int. Cl.: A61K 9/16, A61K 47/06, A61K 47/26, A61K 47/30, A61K 47/38, A61K 31/41

(54) **COMPOSITION OF PRANLUKAST-CONTAINING SOLID PREPARATION WITH IMPROVED BIOAVAILABILITY AND METHOD FOR PREPARING SAME**

(30) Priority: 01.06.2015 KR 20150077587
(71) Applicant: Sama Pharm Co., Ltd., Wonju-si, Gangwon-do 26365 (KR)
(72) Inventor: LEE, Kun-Hee, Suwon-si Gyeonggi-do 16681 (KR); KIM, Su-Jin, Suwon-si Gyeonggi-do 16679 (KR); AHN, Byeong Kil, Suwon-si Gyeonggi-do 16680 (KR); PARK, Sang-Man, Gimhae-si Gyeongsangnam-do 51014 (KR); HWANG, Yong Youn, Suwon-si Gyeonggi-do 16298 (KR)
(74) Representative: Santarelli
(86) International application number: PCT/KR2016/005810
(87) International publication number: WO 2016/195377

(57) **Abstract**

The present invention relates to a pharmaceutical composition containing pranlukast and a method for preparing the same. The present invention has a significantly improved pranlukast release rate and bioavailability compared with conventional pranlukast-containing products.

## Description

### TECHNICAL FIELD

The present invention relates to a preparation containing pranlukast which is an insoluble drug, and a method for preparing the same. More specifically, the present invention relates to a pranlukast-containing solid preparation and a method for preparing the same, which has improved bioavailability as well as release rate by using only the preparation equipment and preparation methods which are commonly used in the field of pharmaceuticals without using any special equipment and processes which causes the manufacturing cost of the preparation to become relatively higher, thereby reducing a single dose of pranlukast as much as possible compared with the products in the market, and improving medication compliance of a patient and reducing side effects resulting from the administration of drugs in a high dose.

### BACKGROUND FIELD

The present invention relates to a pranlukast-containing preparation and a method for preparing the same, which is characterized by reducing the amount of pranlukast in a single dose as much as possible by improving the bioavailability of pranlukast by adopting formulation techniques which are different from the conventional products in the market.

Pranlukast whose chemical name is 4-oxo-8-[4-(4-phenylbutoxy)benzoylamino]-2-(tetrazol-5-yl)-4H-1-benzopyran hemihydrate is a new drug developed by Ono pharmaceutical Co., Ltd. in Japan. Pranlukast is a drug used for treating bronchial asthma and allergic rhinitis by finding out the pharmacological mechanism that pranlukast may selectively bind to leukotriene receptors and make an antagonistic operation. Representative oral pranlukast-containing solid preparations for the adult dosage which are currently available in Korea as of June 2015 include Onon capsules (pranlukast 112.5 mg/cap., two capsules for a single dose, Dong-A ST Co., Ltd.), Pranair capsules (pranlukast 112.5 mg/cap., one capsule for a single dose, SK Chemicals Co., Ltd.) and Prakanon tablets (pranlukast 75 mg/tab., one tablet for a single dose, Yuhan Corporation).

It has been found out that the upper small intestine is where most of the pranlukast is absorbed when oral administration. Pranlukast belongs to Biopharmaceutics Classification System (BCS) Class II, has strong adhesive cohesiveness, and has very low solubility (1.2 µg/ml), and thus has low bioavailability compared with other drugs when orally administered. However, the Onon capsules could not improve the problem of pranlukast in the formulation step, and eventually adopted a manner of increasing the dose of pranlukast. In the case of Onon capsules, it is reported that only when 225 mg of pranlukast is administered in a single dose for adult, said drug is useful for treating the target disease. However, since the amount in a single dose for administration is high, the Onon capsules are not economical, may reduce medication compliance of a patient, and may bring about side effects resulting from the administration of drugs in a high dose.

In order to overcome the above-mentioned disadvantages of the Onon capsules, various researches have been conducted so far.

Korean Patent No. 10-0389606 relates to a spray dried granule with improved adhesive cohesiveness of pranlukast, a method for preparing the same and a method for improving the adhesive cohesiveness of pranlukast. In order to improve the adhesive cohesiveness, the corresponding patent adopted a manner of dissolving saccharide, binders and surfactants in purified water and suspending pranlukast to prepare the spray dried granule.

However, although the spray dried granule disclosed in Korean Patent No. 10-0389606 improved the adhesive cohesiveness of pranlukast, it could not improve the solubility of pranlukast. Accordingly, there are limitations that the spray dried granule has a delayed release and a very low release rate, and thus the bioavailability thereof is still low as a result.

Korean Patent No. 10-1332223 relates to a method for preparing a nano solid dispersion of pranlukast which can increase the bioavailability by improving the low solubility of pranlukast. The pranlukast nano solid dispersion in the corresponding patent has a structure where pranlukast exists in a carrier consisting of polyethylene glycol and poloxamer, and shows improved bioavailability as the drug stably maintains a nano-scale particle size.

However, in order to prepare the nano solid dispersant disclosed in Korean Patent No. 10-1332223, one should go through a mixing process, a hot melt process and a solvent evaporation process. Therefore, there are disadvantages that the preparation cost is high and the preparation process is complex. Additionally, since methanol/dichloromethanol are used as co-solvents, organic solvents may remain in the solid dispersant and the use of organic solvents may cause environmental pollution.

Korean Patent No. 10-1446129 relates to a preparation method which comprises a mixture comprising pranlukast and polypyrrolidone or a polypyrrolidone-vinyl acetate copolymer or a pH adjuster in the mixture. The corresponding patent improves the solubility and release rate by simply mixing pranlukast with hydrophilic polymers. However, the corresponding patent has a high ratio of the hydrophilic polymer compared with active ingredient, poor in-vivo experimental data, and does not suggest any materials proving how the bioavailability has been actually improved.

Korean Patent No. 10-1086254 relates to a pranlukast solid dispersion composition with improved solubility and bioavailability and a method for preparing the same. The corresponding patent comprises a pranlukast solid dispersion composition characterized in that a polyvinyl pyrrolidone vinyl acetate copolymer for pranlukast is prepared by being melt by heat in a weight ratio of 0.2:1-10:1. The Pranair capsules (pranlukast 112.5 mg/cap., one capsule for a single dose, SK Chemicals Co., Ltd.) which are currently in the market are formulations of the solid dispersion disclosed in said patent.

However, although the solid dispersant disclosed in said patent has increased the bioavailability of pranlukast twice compared with the conventional contrast drug, Onon capsules, said solid dispersion cannot be prepared by using the preparation equipment commonly used in the field of pharmaceuticals such as hot melt, which is a method melting by heat. Therefore, there is a burden on equipment investment and the preparation processes are complex. Also, changing the drug to be amorphous by hot melt may be seen as making the drug thermodynamically unstable, and thus the drug may return to a crystal form, which is a stable form within the expiration period of the product. Accordingly, there is a limitation that the solubility and bioavailability of the drug may change.

Korean Patent No. 10-0981751 comprises a tablet of pharmaceutical composition which comprises a pranlukast-containing granule which includes a drug coating layer coated on a granule core. The corresponding patent is characterized in that the suspension of pranlukast, binder and surfactant is coated on a granule core to prepare a granule and make a tablet with these granules. The Prakanon tablets (pranlukast 75 mg/tab., 1 tablet for a single dose, Yuhan Corporation) which are currently in the market are formulations of the spray dried granule disclosed in said patent.

However, the spray dried granule disclosed in said patent needs a suspension process which intensely suspends pranlukast in the solvent, various variables exist in the preparation processes depending on the spray drying process, and the preparation cost may increase due to the complex preparation process. Additionally, although the corresponding patent has improved the bioavailability of pranlukast and reduced the amount of drug in a single dose by 1/3 compared with the conventional contrast drug, the Onon capsule, pranlukast is administered in a single dose of 75mg. Therefore, it is still necessary to improve the bioavailability to reduce the dose of the drug, thereby improving the medication compliance of a patient and reducing side effects resulting from the administration of drugs in a high dose.

### DETAILED DESCRIPTION OF INVENTION

### TECHNICAL PROBLEM

In general, as the drug dosage increases, side effects resulting therefrom would occur more frequently. Therefore, in the case of a drug administered in a high single dose, it is necessary to increase the bioavailability and reduce the dose to reduce the incidence of side effects. However, in spite of various prior arts, the medicine with the smallest single dose for adult among the pranlukast-containing preparations which are currently available in the Korean market is Prakanon tablet (pranlukast 75 mg, Yuhan Corporation), and the corresponding medicine still requires development of a product which can exhibit a clinically equivalent or superior efficacy compared with the conventional medicine in the market even when administered in an amount smaller than 75 mg in a single dose by improving the solubility and release rate of pranlukast and increasing the bioavailability.

The present invention aims to provide a method for preparing solid pranlukast which is an insoluble drug with an improved release rate by using the preparation equipment and methods which are commonly used in the field of pharmaceuticals, and also aims to provide a method which can exhibit a clinically equivalent or superior efficacy compared with products in the market even when administered in an amount smaller than 75 mg in a single dose by increasing the bioavailability of pranlukast.

### TECHNICAL SOLUTION

The present invention achieved the above task by the following means:
(1) A pharmaceutical composition which comprises a granule prepared by a wet granulation method and a pharmaceutically acceptable carrier, characterized in that the wet granulation method uses C₁-C₆ alcohol as a solvent of a binding solution, in that the granule comprises pranlukast, a surfactant, a binder and a diluent, and in that the granule or pharmaceutically acceptable carrier comprises at least two disintegrating agents selected from the group consisting of crospovidone, sodium starch glycolate and croscarmellose sodium.
(2) The composition according to (1) above, characterized in that the diluent is at least one selected from the group consisting of lactose and microcrystalline cellulose.
(3) The composition according to (1) or (2) above, characterized in that the binder is at least one selected from the group consisting of polyvinyl pyrrolidone, polyvinyl pyrrolidone-vinyl acetate copolymer and hydroxypropyl methylcellulose.
(4) The composition according to any one of (1) to (3) above, characterized in that the surfactant is at least one selected from the group consisting of polyethylene glycol-15-hydroxystearate, polyoxyethylene-polyoxypropylene copolymer, polyoxyethylene sorbitan fatty acid ester and sodium lauryl sulfate.
(5) The composition according to any one of (1) to (4) above, characterized in that pranlukast is administered in an amount of 70 mg or less.
(6) The composition according to any one of (1) to (5) above, characterized in that the diluent is contained in an amount of 20-60% of the total weight of the composition.
(7) The composition according to any one of (1) to (6) above, characterized in that the binder is contained in an amount of 2-10% of the total weight of the composition.
(8) The composition according to any one of (1) to (7) above, characterized in that the surfactant is contained in an amount of 5-20% of the total weight of the composition.
(9) A preparation comprising the pharmaceutical composition according to any one of (1) to (8) above, characterized in that the preparation is a granule, a tablet, an oral disintegrating agent, a chewable tablet, a dispersible tablet, a capsule, a fine granule or a dry syrup.

### EFFECT OF INVENTION

According to the present invention, the release rate of pranlukast is improved, and the bioavailability thereof is remarkably improved compared with the conventional product in the market, the Onon capsule, and accordingly an efficacy clinically equivalent or superior to the Onon capsule (pranlukast 112.5 mg/cap., two capsules for a single dose, Donga-ST Co., Ltd.) can be exhibited with the administration of 70 mg or less of pranlukast in a single dose.

### BRIEF DESCRIPTION OF DRAWINGS

Fig. 1 represents a result of a dissolution test of examples 11, 14 and 16 and the Onon capsule in an eluate of pH 6.8;
Fig. 2 represents a result of a dissolution test of examples 12 to 15 in an eluate of pH 4.0+2% PSB 80 (polysorbate 80);
Fig. 3 represents a result of a dissolution test of examples 14 and 17 and the Onon capsule in an eluate of pH 4.0+2% PSB 80 (polysorbate 80);
Fig. 4 represents a result of a pharmacokinetics test of a tablet which contains a granule prepared by a preparation method according to the present invention (example 12) and the Onon capsule in a human being;
Fig. 5 represents a result of a pharmacokinetics test of a tablet which contains a granule prepared by a preparation method according to the present invention (example 14) and the Onon capsule in a human being; and
Fig. 6 represents a result of a pharmacokinetics test of a tablet which contains a granule prepared by a preparation method according to the present invention (example 17) and the Onon capsule in a human being.

### BEST MODE FOR EMBODIMENT OF THE INVENTION

When formulating drugs, a granule may be prepared as a pre-treatment process in order to secure mobility of particulate materials, increase the density of raw materials, and improve powder compressibility during tableting, etc.

There are wet methods and dry methods as a method for preparing granules, and the present invention uses a wet method. Specially, the granule according to the present invention is obtained by mixing pranlukast with a diluent in a high-speed mixer which is commonly used in the field of pharmaceuticals, suspending a binder and a surfactant in an alcohol solvent, kneading and drying with the corresponding binding solution.

Surprisingly, the present inventors found out that when preparing the pranlukast granule in a high-speed mixer by using the wet method, the following advantages could be obtained compared with the hot melt manner and the spray dry manner which are the manners of the conventional products in the market for improving the release rate and bioavailability.

According to the preparation method of the Pranair capsule (pranlukast 112.5 mg/cap., one capsule for a single dose, SK Chemicals Co., Ltd.) which is a formulation of the solid dispersion disclosed in Korean Patent No. 10-1086254, pranlukast and polymer are melted with heat in a state where the temperature is raised to 150-200°C by using a melt-flow-indexer. However, since the melt-flow-indexer used for preparing the solid dispersion is not an equipment generally used in the field of pharmaceuticals, the preparation costs resulting from equipment investment would increase. Additionally, since pranlukast and polymer are melted with heat at 150-200°C, this method cannot be applied when the active ingredients and excipients are unstable under heat. Especially, the corresponding final formulation is in the form of a capsule, and thus is weak to temperature and humidity compared with a tablet, and the capsule material may give a negative influence on the dissolution of drugs and in-vivo release.

The preparation method of the Prakanon tablet (pranlukast 75 mg/tab., one tablet for single dose, Yuhan Corporation) which is a formulation of the spray dried granule disclosed in Korean Patent No. 10-0981751 is characterized by coating a suspension of pranlukast, a water-soluble polymer and a surfactant on a granule core to prepare the granule and conducting tableting by using the same.

However, in the above preparation process, due to the low solubility of pranlukast, an intense dispersing process, for example, a dispersion process using a propeller-containing mixer, a homogenizer or an ultrasonic oscillator, etc. is necessary when suspending pranlukast in the solvent, which causes an increase in the preparation time. Also, since a spray drying process should be conducted, the preparation costs would be high and the production process would be complex.

However, in the case of the preparation process according to the present invention, since the granule is prepared simply with a high-speed mixer which is commonly used in the field of pharmaceuticals, no additional investment on equipment is necessary. Also, since a simple preparation process was adopted, it is not difficult to apply this process to the pharmaceutical industry. Furthermore, there is no process of suspending pranlukast in a solvent, a process of dispersing pranlukast is unnecessary, and thus the preparation time would be shortened.

When preparing the granule according to the present invention, diluents which are commonly used in the field of pharmaceuticals may be used. For example, a diluent selected from at least one of xylitol, mannitol, isomalt, sorbitol, maltitol, purified white sugar, lactose, inositol, erythritol, crystalline fructose, trehalos, ribitol, arabitol, galactitol, lactitol, maltotritol, microcrystalline cellulose, sodium cross carboxymethyl cellulose, calcium carboxymethyl cellulose, hydroxypropyl cellulose, sodium carboxymethyl cellulose, carboxymethyl cellulose, gelatinized starch, glucose, pre-gelatinized starch, starch, corn starch, light silicic acid anhydride or crystalline cellulose, etc. may be used. Especially, microcrystalline cellulose, lactose or a mixture thereof are preferable in terms of exhibiting the target effect of the present invention. It is preferable for the diluent to be contained in an amount of 20-60% of the total weight of the pharmaceutical composition in terms of exhibiting the target effect of the present invention.

When preparing the granule according to the present invention, binders which are commonly used in the field of pharmaceuticals may be used. For example, a binder selected from at least one of polyvinyl pyrrolidone, polypyrrolidone/vinyl acetate copolymer, hydroxypropyl methylcellulose, hydroxypropyl cellulose, polyethylene glycol, polyvinyl alcohol, etc. may be used. Especially, at least one selected from the group consisting of polyvinyl pyrrolidone, polypyrrolidone/vinyl acetate copolymer, and hydroxypropyl methylcellulose is preferable in terms of exhibiting the target effect of the present invention, and among the above, polyvinyl pyrrolidone is the most preferable. It is preferable for the binder to be contained in an amount of 2-10% of the total weight of the pharmaceutical composition in terms of exhibiting the target effect of the present invention.

When preparing the granule according to the present invention, a surfactant selected from at least one of polyethylene glycol-15-hydroxystearate (for example, Kolliphor® HS 15), polyoxyethylene glycol natural or hydrogenated castor oil (for example, Kolliphor® RH 40), polyoxyethylene-polyoxypropylene copolymer (for example, poloxamers), polyoxyethylene sorbitan fatty acid ester (for example, polysorbates), sodium lauryl sulfate, glyceryl fatty acid ester (for example, glyceryl monostearate), etc. may be used. Especially, at least one selected from the group consisting of polyethylene glycol-15-hydroxystearate, polyoxyethylene-polyoxypropylene copolymer, polyoxyethylene sorbitan fatty acid ester and sodium lauryl sulfate is preferable in terms of exhibiting the target effect of the present invention. It is preferable for the surfactant to be contained in an amount of 5-20% of the total weight of the pharmaceutical composition in terms of exhibiting the target effect of the present invention.

When preparing the granule according to the present invention, C₁-C₆ alcohol is a preferable alcohol solvent in terms of exhibiting the target effect of the present invention. For example, low alcohols such as methanol, ethanol, propanol, isopropanol, butanol, etc. may be used. Especially, when using ethanol, bioavailability of pranlukast was improved remarkably.

The pharmaceutical composition according to the present invention comprises a solid granule of pranlukast and a pharmaceutically acceptable carrier.

The pharmaceutically acceptable carrier includes excipients, disintegrating agents, lubricants, etc. which are already known and used. The pharmaceutically acceptable carriers which can be used in the present invention may be at least one of xylitol, mannitol, isomalt, sorbitol, maltitol, purified white sugar, lactose, inositol, erythritol, crystalline fructose, trehalos, ribitol, arabitol, galactitol, lactitol, maltotritol, microcrystalline cellulose, sodium cross carboxymethyl cellulose, calcium carboxymethyl cellulose, hydroxypropyl cellulose, sodium carboxymethyl cellulose, carboxymethyl cellulose, gelatinized starch, glucose, pre-gelatinized starch, starch, corn starch, light silicic acid anhydride or crystalline cellulose, or a mixture thereof, etc. Additionally, disintegrating agents may be at least one of sodium starch glycolate, croscarmellose sodium, crospovidone and low substituted hydroxypropyl cellulose, etc. Lubricants may be at least one of sodium stearyl fumarate, magnesium stearate, stearic acid, colloidal silicon dioxide, etc.

Especially, pranlukast is mostly absorbed in the upper small intestine, and the amount of pranlukast absorbed intends to decrease gradually as pranlukast goes down to the lower small intestine and large intestine. Accordingly, the present inventors conceived that only when the disintegration of pharmaceutical formulations (tablets or capsules, etc.) is completed before reaching the upper small intestine where most of the drug is absorbed, a great amount of drug would be absorbed in the corresponding topical absorption area, and intended to design a formulation which can be disintegrated fast before reaching the upper small intestine. As a result of numerous repeated experiments for this, it was found out that the target effect of the present invention can be achieved when comprising at least two types of disintegrating agents selected from the group consisting of crospovidone, sodium starch glycolate and croscarmellose sodium. The disintegrating agent may be mixed with the granule, or may be mixed with the carrier, not with the granule.

The pharmaceutically acceptable carrier may be flexibly adjusted with respect to the total weight of the composition, and can be properly selected according to the formulation which is finally obtained.

The final formulation of the pharmaceutical composition according to the present invention may have variety forms, but preferably may be granules, tablets, oral disintegrating agents, chewable tablets, suspension tablets, capsules, fine granules or dry syrup, etc. These formulations may be prepared according to a method which is commonly used in the field of pharmaceuticals. For example, tablets may be prepared by mixing the granules according to the present invention with excipients, disintegrating agents, lubricants, etc. and tableting the same. Additionally, a film coating process may be added for improving stability of drug and convenience of administration, differentiating in appearance, etc.

As can be confirmed from the following examples and experimental examples, the pharmaceutical formulation according to the present invention can exhibit a clinically equivalent or superior efficacy even when administered in a dose about 4.5 times lower than a single dose of the conventional control product in the market, Onon capsule (pranlukast 112.5 mg/cap., two capsules for a single dose, Donga-ST Co., Ltd.) by improving the release rate and bioavailability of pranlukast. Additionally, the pharmaceutical formulation according to the present invention can exhibit a clinically equivalent or superior efficacy even when administered in a dose lower than Prakanon tablet (pranlukast 75 mg/tab., one tablet for a single dose, Yuhan Corporation) which lowered the single dose as much as possible among the conventional products for improving bioavailability. In other words, even if the pharmaceutical composition according to the present invention contains pranlukast in an amount of about 70 mg or less, said composition exhibits a clinically equivalent or superior efficacy compared with the conventional pranlukast-containing products for a single dose. This satisfies the necessity for developing products which may exhibit a clinically equivalent or superior efficacy compared with the conventional medicines in the market while administering a small amount of the drug and reducing the incidence of side effects, which is required in the industry.

Hereinafter, the present invention will be explained through the examples below. The examples below do not limit the present invention in any manner.

### EMBODIMENT OF THE INVENTION

### [EXAMPLES]

### [EXAMPLE 1-10] Preparation of pranlukast wet granule

The active ingredient (pranlukast) and a diluent were put in a high-speed mixer and mixed. A binder and a surfactant were stirred in the solvent and dissolved to obtain a binding solution. The binding solution was put in the mixture and was kneaded in the high-speed mixer. A granulation process using a sieve may be added as necessary, and the solvent is evaporated with a tray drier. The dried material was sieved with a sieve to obtain pranlukast wet granule. A specific composition thereof is as shown in Table 1 below.

**[Table 1]**

| Purpose of mixture | Active ingredie nt | Diluent | | Binder | | | Surfactant | | | | Solvent | |
|---|---|---|---|---|---|---|---|---|---|---|---|---|
| Name of ingredient | pranlukast | lactose | Microcr ystalline cellulose | polyvinyl pyrrolidone | Polypyrrol idone/vinyl acetate copolymer | hydroxypropylmethylcellulose | polyethylene glycol-15-hydroxystearate | polyoxy ethylene - polyoxy propylene copolymer | polyoxyeth ylenesorbit an fatty acid ester | sodium lauryl sulfate | ethanol | water |
| Example 1 | 105g | 307.5 g | - | 22.5g | - | - | 24g | - | - | - | - | 60g |
| Example 2 | 105g | - | 271.5g | 22.5g | - | - | 24g | 43.5g | - | - | - | 150g |
| Example 3 | 105g | - | 279g | - | 22.5g | - | 24g | 43.5g | - | - | - | 150g |
| Example 4 | 105g | 129g | 129g | - | - | 12g | 24g | 43.5g | - | - | | 150g |
| Example 5 | 105g | - | 279g | 22.5g | - | - | 24g | 43.5g | - | - | 180g | - |
| Example 6 | 105g | 129g | 129g | 22.5g | - | - | 24g | 43.5g | - | - | 120g | - |
| Example 7 | 105g | 129g | 129g | 22.5g | - | - | 24g | - | 43.5g | - | 120g | - |
| Example 8 | 105g | 129g | 129g | 22.5g | - | - | - | 43.5g | 24g | - | 120g | - |
| Example 9 | 105g | 129g | 129g | 22.5g | - | - | - | 43.5g | - | 24g | 120g | - |
| Example 10 | 100g | 122g | 122g | 22g | - | - | 24g | 42g | - | - | 100g | - |

### [EXAMPLE 11-17] Preparation of pranlukast-containing tablet

A tablet was prepared by using the granule prepared in examples 1, 6, 8 and 10 with the ingredients in the contents shown in Table 2 below. Specifically, a wet granule and a disintegrating agent were put in the mixer and mixed, and the lubricant was put and further mixed. Afterwards, examples 11-17 were prepared by going through tableting and coating. Examples 11-16 were prepared to contain 70 mg of pranlukast for each tablet, and example 17 was prepared to contain 50 mg of pranlukast for each tablet.

**[Table 2]**

| | | Examples | | | | | | |
|---|---|---|---|---|---|---|---|---|
| | | Example 11 | Example 12 | Example 13 | Example 14 | Example 15 | Example 16 | Example 17 |
| Granules | Example 1 | 306 mg | - | - | - | - | - | |
| | Example 6 | - | 302 mg | 302 mg | 302 mg | 302 mg | | |
| | Example 8 | - | - | - | - | - | 302 mg | |
| | Example 10 | - | - | - | - | - | - | 216 mg |
| Disintegrating agents | crospovido ne | 11.5 mg | 11 mg | 16 mg | 16 mg | - | 11 mg | 16 mg |
| | sodium starch glycolate | 9.5 mg | 9 mg | 16mg | - | 16 mg | 9 mg | - |
| | croscarmel lose sodium | - | - | - | 16 mg | 16 mg | - | 16 mg |
| lubricants | colloidal silicon dioxide | 7 mg | 7 mg | 7 mg | 7 mg | 7 mg | 7 mg | 5 mg |
| | magnesiu m stearate | 9 mg | 9 mg | 9 mg | 9 mg | 9 mg | 9 mg | 7 mg |
| Coating agents | opadry | 12 mg | 12 mg | 12 mg | 12 mg | 12 mg | 12 mg | 10 mg |
| Total weight | | 355 mg | 350 mg | 362 mg | 362 mg | 362 mg | 350 mg | 270 mg |

### [EXPERIMENTAL EXAMPLE 1] Comparative dissolution test

A comparative dissolution test was conducted according to the method 2 dissolution test of the Korean Pharmacopoeia for one tablet prepared in each of examples 11, 14 and 16 and two Onon capsules (pranlukast 112.5 mg/cap., two capsules for a single dose, Donga-ST Co., Ltd.).

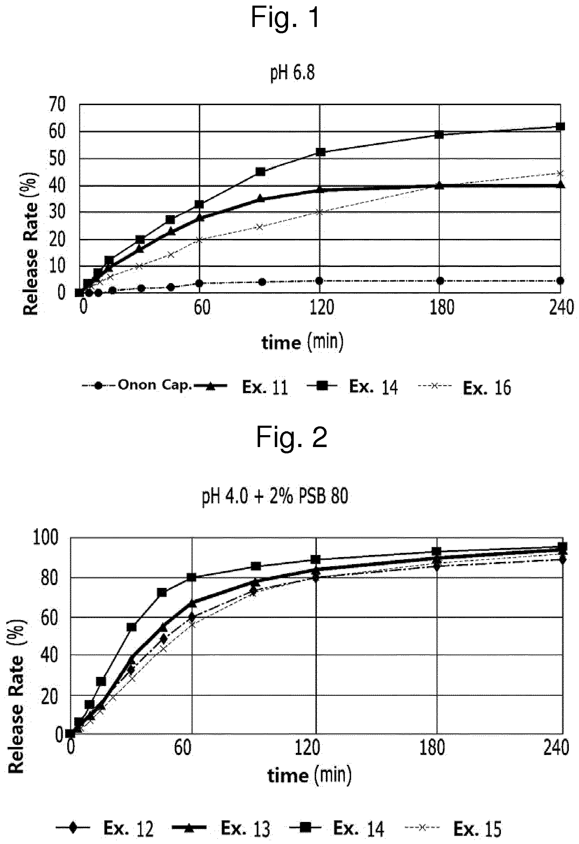

900 mL of solutions of pH 6.8 were respectively put in dissolution vessels and were stirred at a rotation speed of 50 rpm while maintaining a temperature of 37±0.5°C to measure the release rate. At each point, about 5 ml of the solution was taken, filtered with a 0.45 µm filter and analyzed with HPLC. The result thereof is as shown in Fig. 1 below.

As can be seen from Fig. 1, the release rate of the tablet prepared from the wet granule according to the present invention increased remarkably compared with the Onon capsule, which is a preparation available in the market.

### [EXPERIMENTAL EXAMPLE 2] Comparative dissolution test

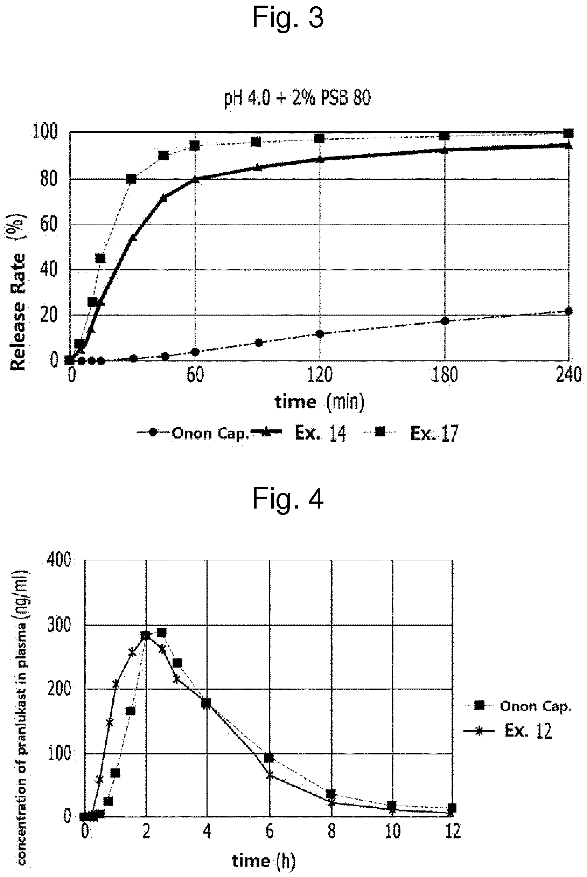

A comparative dissolution test was conducted according to the method 2 dissolution test of the Korean Pharmacopoeia for one tablet prepared in each of examples 12-15 and 17 and two Onon capsules (pranlukast 112.5 mg/cap., two capsules for the single dose, Donga-ST Co., Ltd.). 900 mL of solutions of pH 4.0 containing 2% PSB 80 (polysorbate 80) were put in the dissolution vessels and were stirred with a rotation speed of 50 rpm while maintaining a temperature at 37±0.5°C to measure the release rate. At each point, about 5 ml of the solution was taken, filtered with a 0.45 µm filter and analyzed with HPLC. The result thereof is as shown in Figs. 2 and 3 below.

As can be seen from Fig. 2, it could be confirmed that the initial release rate may vary depending on the type of the disintegrating agent and the amount of disintegrating agent input.

As can be seen from Fig. 3, it could be confirmed that the initial release rate of example 17 increased than that of example 14, and that the overall release rate of examples greatly increased compared with the Onon capsule.

### [EXPERIMENTAL EXAMPLE 3] Evaluation of pharmacokinetics in a human being

The pharmacokinetics in a human being were evaluated by using the tablet prepared according to the present invention (example 12) and the Onon capsule (two capsules administered) which is a preparation in the market.

Twelve healthy adults aged between 20 and 40 were divided into two groups (n=6), and the tablet prepared in example 12 (example 12, one tablet, pranlukast 70 mg) (first group) and the preparation in the market (two Onon capsules, total amount of pranlukast 225 mg) (second group) were cross-administered together with 150 ml of water on an empty stomach. The drug-free interval was set as one week. About 10 mL of blood was drawn right before administration and 0.25, 0.5, 0.75, 1, 1.5, 2, 2.5, 3, 4, 6, 8 and 12 hours after the administration. The drawn blood was put in a vacuum tube treated with heparin, roller-mixed for 5 minutes, and centrifuged for 5 minutes at 3000 rpm. Plasma was taken and put in an Eppendorf tube and frozen at -70°C before the analysis. The concentration of pranlukast in the plasma was analyzed using LC/MS. The result of the analysis is as shown in Table 3 and Fig. 4 below.

**[Table 3]**

| | AUCt | Cmax |
|---|---|---|
| Example 12 (one tablet) | 1069.4 ng/ml | 282.1 ng/ml |
| Preparation in the market (two Onon capsules) | 1025.2 ng/ml | 302.1 ng/ml |
| T/R Ratio | 104.3% | 93.3% |

It can be confirmed that the tablet in example 12 prepared from the wet granule according to the present invention can exhibit a clinically equivalent efficacy even when administered in a dose far less (70mg/tab.) than a total 225 mg of pranlukast for the two Onon capsules, which are conventional products in the market.

### [EXPERIMENTAL EXAMPLE 4] Evaluation of pharmacokinetics in a human being

The pharmacokinetics in a human being were evaluated by using the tablet prepared according to the present invention (example 14) and the Onon capsule (two capsules administered) which is a preparation in the market.

The test was conducted in the same manner as experimental example 3 except for the drug tested.

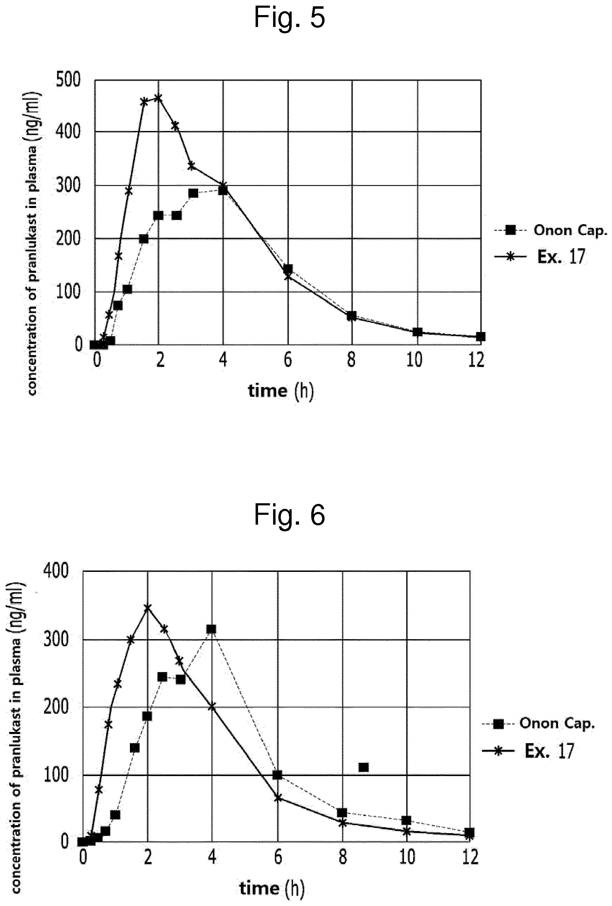

The result thereof is as shown in Table 4 and Fig. 5 below.

**[Table 4]**

| | AUCt | Cmax |
|---|---|---|
| Example 14 (one tablet) | 1924.1 ng/ml | 500.2 ng/ml |
| Preparation in the market (two Onon capsules) | 1393.1 ng/ml | 375.9 ng/ml |
| T/R Ratio | 138.1% | 133.0% |

The tablet in example 14 prepared from the wet granule according to the present invention exhibited a result with an AUCt and Cmax value respectively about 38% and 33% higher than the preparation in the market even when administered in a dose far less (70 mg/tab.) than a total 225 mg of pranlukast for the two Onon capsules, which are conventional products in the market. Therefore, it could be confirmed that since the tablet prepared from the wet granulate according to the present invention provides an improved bioavailability, said tablet can exhibit a clinically equivalent efficacy compared with the preparation in the market even when administered in a single dose (about 50 mg) lower than 70 mg.

### [EXPERIMENTAL EXAMPLE 5] Evaluation of pharmacokinetics in a human being

The pharmacokinetics in a human being were evaluated by using the tablet prepared according to the present invention (example 17) and the Onon capsule (two capsules administered) which is a preparation in the market.

The test was conducted in the same manner as experimental example 3 except for the drug tested.

The result thereof is as shown in Table 5 and Fig. 6 below.

**[Table 5]**

| | AUCt | Cmax | Tmax |
|---|---|---|---|
| Example 17 (one tablet) | 1230.2 ng/ml | 353.0 ng/ml | 2.06 hr |
| Preparations in the market (two capsules) | 1171.9 ng/ml | 384.0 ng/ml | 3.02 hr |
| T/R Ratio | 105.0% | 91.9% | 63.2% |

The tablet in example 17 prepared from the wet granule according to the present invention exhibited an equivalent level of AUCt, Cmax and T/R Ratio values even when administered in a dose far less (50 mg/tab.) than a total 225 mg of pranlukast for the two Onon capsules, which are conventional products in the market. Therefore, it could be confirmed that said table can exhibit a clinically equivalent efficacy compared with the preparation in the market. Additionally, since the result shows that the Tmax value is faster than the conventional preparations in the market, said tablet is expected to exhibit the efficacy even faster for patients suffering from asthma and allergic rhinitis, which are indications of pranlukast. Therefore, it could be confirmed that said tablet even has improved therapeutically compared with the conventional preparation in the market.

### INDUSTRIAL APPLICABILITY

The present invention relates to a pharmaceutical composition which has pranlukast as an active ingredient and comprises a granule prepared from a wet granulation method, characterized in that the wet granulation method uses C₁-C₆ alcohol as a solvent of a binding solution, and the granulation comprises pranlukast, a surfactant, a binder and a diluent. The product of the present invention is a drug which has an efficacy equivalent or superior to the conventional products in the market even when administering pranlukast in a single dose of 50 ml by remarkably improving the release rate of the drug compared with the conventional Onon capsule, and improving the bioavailability of the drug by about 4.5 times as a result of conducting a pharmacokinetics test on a human being.

Additionally, the preparation method of the present invention is characterized by adopting the wet granulation method which is commonly used in the field of pharmaceuticals compared with the conventional products for improving bioavailability of pranlukast and related patent technologies. Said preparation method has an advantage that there is substantially no difficulty in applying the method to the industry because the method has a simple preparation process and can prevent the increase of preparation cost.

## Claims

1. A pharmaceutical composition comprising a granule prepared by a wet granulation method, **characterized in that** the wet granulation method uses C₁-C₆ alcohol as a solvent of a binding solution, **in that** the granule comprises pranlukast, a surfactant, a binder and a diluent, and **in that** the pharmaceutical composition comprises at least two disintegrating agents selected from the group consisting of crospovidone, sodium starch glycolate and croscarmellose sodium.

2. The composition according to claim 1, **characterized in that** the diluent is at least one selected from the group consisting of lactose and microcrystalline cellulose.

3. The composition according to claim 1, **characterized in that** the binder is at least one selected from the group consisting of polyvinyl pyrrolidone, polyvinyl pyrrolidone-vinyl acetate copolymer and hydroxypropyl methylcellulose.

4. The composition according to claim 1, **characterized in that** the surfactant is at least one selected from the group consisting of polyethylene glycol-15-hydroxystearate, polyoxyethylene-polyoxypropylene copolymer, polyoxyethylene sorbitan fatty acid ester and sodium lauryl sulfate.

5. The composition according to claim 1, **characterized in that** pranlukast is administered in a single dose of 70 mg or less.

6. The composition according to claim 1, **characterized in that** the diluent is contained in an amount of 20-60% of the total weight of the composition.

7. The composition according to claim 1, **characterized in that** the binder is contained in an amount of 2-10% of the total weight of the composition.

8. The composition according to claim 1, **characterized in that** the surfactant is contained in an amount of 5-20% of the total weight of the composition.

9. A preparation comprising the pharmaceutical composition according to any one of claims 1 to 8, **characterized in that** the preparation is a granule, a tablet, an oral disintegrating agent, a chewable tablet, a dispersible tablet, a capsule, a fine granule or a dry syrup.
